# EUROPEAN PATENT SPECIFICATION

(11) **EP 4 284 356 B1**
(45) Date of publication and mention of the grant of the patent: **02.04.2025**
(21) Application number: 22708379.7
(22) Date of filing: 28.01.2022
(51) Int. Cl.: A61K 31/216, A61P 25/00, A61K 31/4422, A61K 31/517

(54) **COMPOUNDS FOR USE IN THE TREATMENT OF ATTENTION DEFICIT HYPERACTIVITY DISORDER**
VERBINDUNGEN ZUR VERWENDUNG IN DER BEHANDLUNG VON AUFMERKSAMKEITSDEFIZIT-/HYPERAKTIVITÄTSSTÖRUNG
COMPOSÉS POUR L'UTILISATION DANS LE TRAITEMENT DU TROUBLE DU DÉFICIT DE L'ATTENTION AVEC HYPERACTIVITÉ

(30) Priority: 29.01.2021 GB 202101291; 29.01.2021 GB 202101292; 29.01.2021 GB 202101296
(43) Date of publication of application: 06.12.2023
(73) Proprietor: 3Z EHF, 102 Reykjavik (IS)
(72) Inventor: KARLSSON, Karl Ægir, Reykjavik 102 (IS); ÞORSTEINSSON, Haraldur, Reykjavik 102 (IS)
(74) Representative: Abel & Imray LLP
(86) International application number: PCT/EP2022/052142
(87) International publication number: WO 2022/162199

(56) References cited:
- WO-A1-2004/002487
- WO-A1-2005/123073
- WO-A1-2014/133329
- WO-A1-2019/081691
- WO-A1-2020/120606
- WO-A1-99/25364
- KR-A- 20140 135 676
- US-A1- 2008 226 715
- DATABASE BIOSIS [online] BIOSCIENCES INFORMATION SERVICE, PHILADELPHIA, PA, US; July 2012 (2012-07-01), TOAL COREY B ET AL: "Long-acting dihydropyridine calcium-channel blockers and sympathetic nervous system activity in hypertension: A literature review comparing amlodipine and nifedipine GITS", Database accession no. PREV201200565844
- BLOOD PRESSURE, vol. 21, no. Suppl. 1, July 2012 (2012-07-01), pages 3 - 10, ISSN: 0803-7051(print), DOI: 10.3109/08037051.2012.690615
- DATABASE MEDLINE [online] US NATIONAL LIBRARY OF MEDICINE (NLM), BETHESDA, MD, US; April 1997 (1997-04-01), HIEBLE J P ET AL: "Recent advances in the identification of alpha1- and alpha2-adrenoceptor subtypes: therapeutic implications.", Database accession no. NLM15989605
- HIEBLE J P ET AL: "Recent advances in the identification of alpha1- and alpha2-adrenoceptor subtypes: therapeutic implications.", EXPERT OPINION ON INVESTIGATIONAL DRUGS APR 1997, vol. 6, no. 4, April 1997 (1997-04-01), pages 367 - 387, ISSN: 1744-7658
- TOAL COREY B ET AL: "Long-acting dihydropyridine calcium-channel blockers and sympathetic nervous system activity in hypertension: A literature review comparing amlodipine and nifedipine GITS", BLOOD PRESSURE, vol. 21, no. Suppl. 1, July 2012 (2012-07-01), pages 3 - 10, XP002807160, ISSN: 0803-7051
- HIEBLE J P ET AL: "Recent advances in the identification of alpha1- and alpha2-adrenoceptor subtypes: therapeutic implications.", EXPERT OPINION ON INVESTIGATIONAL DRUGS APR 1997, vol. 6, no. 4, April 1997 (1997-04-01), pages 367 - 387, XP000981272, ISSN: 1744-7658

## Description

### Field of the Invention

The present invention relates to the treatment and/or prophylaxis of Attention Deficit/Hyperactivity Disorder (ADHD). The present invention also relates to dosage regimens and kits that find utility in the treatment and/or prophylaxis of ADHD.

### Background of the Invention

Attention Deficit/Hyperactivity Disorder (ADHD) is characterised by impaired levels of attention, hyperactivity or impulsivity, or a combination thereof. It is estimated that the condition affects approximately 5% of individuals under the age of 18 years worldwide with persistence of symptoms into adulthood in approximately 65% of cases. The prevalence of ADHD in adults is estimated to be approximately 2.5% worldwide (Thapar & Cooper, The Lancet, 2016, 387(10024), 1240-1250).

The aetiology of ADHD is complex and not fully understood. However, impairment of dopaminergic neurotransmission is considered to be a common feature in ADHD patients. Recent studies have also found that genetic mutations in the *Latrophilin 3* (*LPHN3*, also referred to as *ADGRL3*) gene are strongly associated with ADHD (Arcos-Burgos et al., 2010, Molecular Psychiatry, 15, 1053-1066). Further studies in zebrafish have shown that down regulation of *latrophilin3.1* (*lphn3.1*), the zebrafish *LPHN3* homologue, results in hyperactivity (Lange et al. Mol Psychiatry 2012, 17, 946-954 and Lange et al., 2018, Prog Neuropsychopharm Biol Psych, 84, 181-189). These studies also suggest that down regulation of *lphn3.1* is linked to aberrant dopaminergic neurotransmission.

Established treatments for ADHD include pharmacological treatments such as stimulants (for example, methylphenidate, dexamphetamine and lisdexamfetamine), norepinephrine reuptake inhibitors (for example, atomoxetine) and α2-adrenergic agonists (for example, guanfacine and clonidine). Treatment of ADHD may also include the use of non-pharmacological therapies either as a monotherapy or in combination with pharmacological treatments. Examples of non-pharmacological treatments that may benefit patients with ADHD include cognitive behavioural therapy (CBT), dietary treatments (for example, supplementary fatty acids and the exclusion of artificial food colour), and exercise programs.

Clinical guidelines such as the UK National Institute for Health and Care Excellence (NICE) guidelines recommend the use of methylphenidate, atomoxetine and dexamphetamine for the treatment of ADHD in children or adolescents, and lisdexamfetamine or methylphenidate for the treatment of ADHD in adults. However, despite the approved use of such treatments for ADHD, their use remains controversial due to their limited efficacy and/or adverse effects (Cortese et al., 2018, The Lancet, Psychiatry, 5(9), 727-738).

KR 2014-0135676 discloses the T-type calcium channel blockers useful for the treatment of ADHD: ethosuximide, mibefradil, tetramethrin, SUN-N8075, eponidipine, Y3+, La3+, Ce3+ or Nd3+.

Thus, there remains a need for further treatments for ADHD that provide clinical benefits whilst also displaying good clinical tolerability.

### Summary of the Invention

The present invention provides a compound according to formula (II) 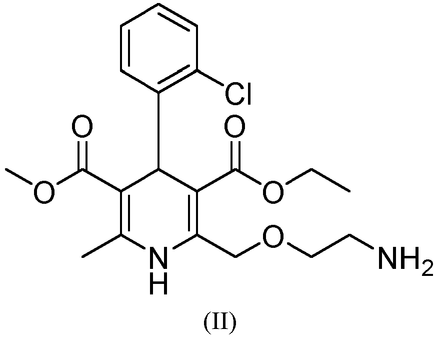 or a pharmaceutically acceptable salt or solvate thereof; for use in the treatment or prophylaxis of Attention Deficit/Hyperactivity Disorder (ADHD).

The present invention further provides the compound of formula (II) for use in a method for the treatment and/or prophylaxis of ADHD, comprising a step of administering a dose of the compound of formula (I), (II) or (III) to a patient known to have, suspected of having, or at risk of developing ADHD.

The present inventors have found that in a zebrafish (*Danio rerio*) model of ADHD, the compound of formula (II) was surprisingly effective at reducing ADHD symptoms such as hyperactivity and motor impulsivity in *Lphn3.1* knock-out zebrafish larvae.

The present invention further provides a kit comprising the compound according to formula (II) and one or more further pharmacological interventions, instructions for a dietetic intervention and/or instructions for a psychological intervention. The kit of the present invention finds use in the treatment or prophylaxis of ADHD.

### Description of the Drawings

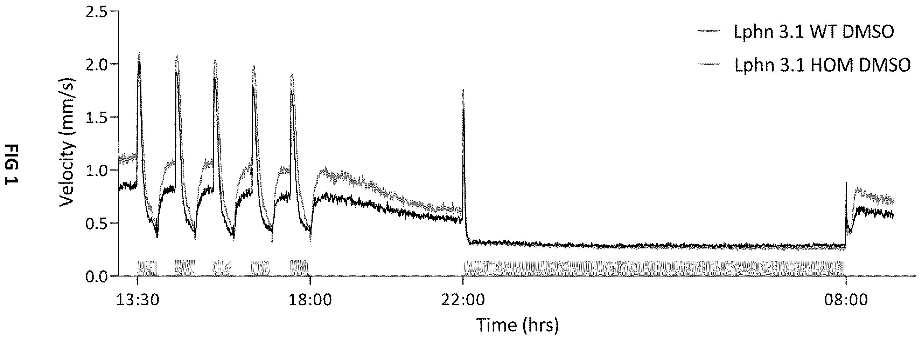

Figure 1 shows a spectrogram of a 24 hour recording of the velocity (mm/s) of zebrafish larvae with a homozygous knock-out of the *Lphn3.1* gene (herein referred to as "Lphn3.1 HOM") and zebrafish larvae with a wild-type *Lphn3.1* gene (herein referred to as "Lphn3.1 WT"). Both groups of larvae were administered a vehicle control (i.e. DMSO only). The grey area shown on the spectrogram indicates the lights-off phases of the experiment (five 30 minute phases, the first phases starting at 1.30 pm and followed by a 10 hour night period wherein lights were off, starting at 10:00 p.m. and ending at 8:00 a.m. the morning after).

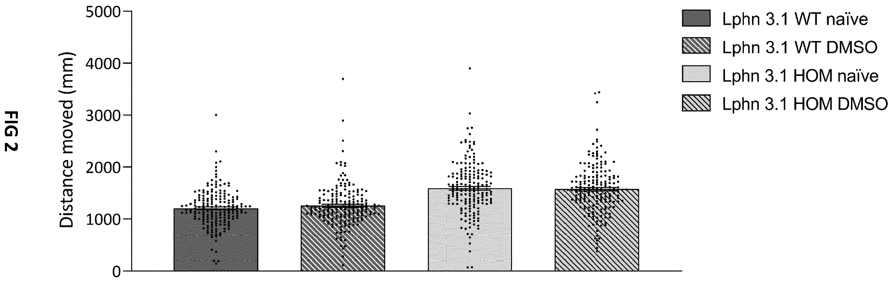

Figure 2 shows a bar graph that depicts the differences in average distance moved by Lphn3.1 HOM and Lphn3.1 WT larvae during the five 30 minute lights-on phases. Non-significant interaction was observed between the genotype and vehicle (i.e. DMSO) (f=1,003, df=1, p= 0.317). A significant effect of genotype on distance moved, between Lphn3.1 HOM and Lphn3.1 WT larvae, was observed (f=117,67, df=1, p<.001). A non-significant effect of the vehicle was demonstrated between naive (i.e. larvae that received neither a test compound or a vehicle control) and DMSO treated larvae (f=0,222 df=1, p=0.638).

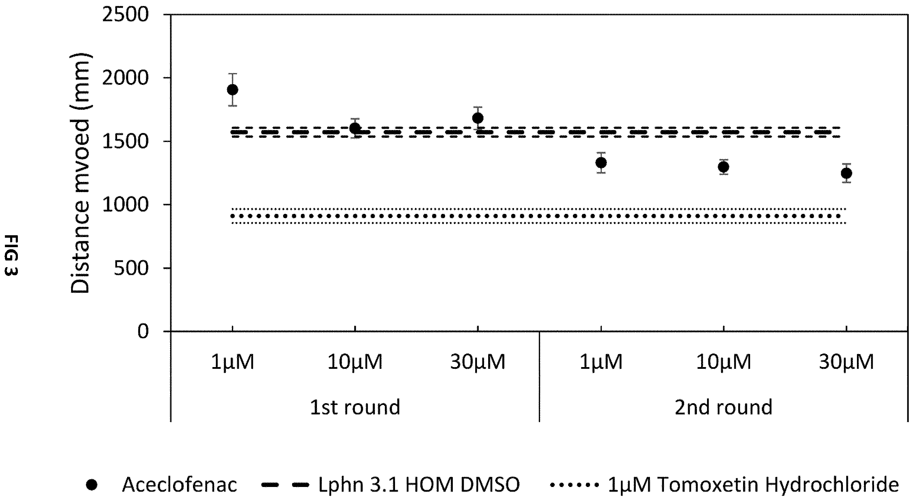

Figure 3 shows a comparison of the distance moved by Lphn3.1 HOM larvae that received aceclofenac, atomoxetine (referred to as tomoxetin hydrochloride in Figure 3) or a vehicle control (i.e. DMSO only). The dashed line on the plot indicates the average distance moved by Lphn3.1 HOM larvae over five lights-on phases following treatment with the vehicle control (n=189). The dotted line indicates the average distance moved by Lphn3.1 HOM larvae over the five lights-on phases following treatment with 1 µM atomoxetine hydrochloride (n=24). The dots indicate the effect of aceclofenac at a dosage of 1 µM, 10 µM or 30 µM on the average distance moved by Lphn3.1 HOM larvae over the five lights-on phases (n=24) (all data presented with +/- SEM). Statistically significant differences were found between larvae treated with aceclofenac and larvae that received the vehicle control (f=3,479, df=3, p=.017, second round: f=7,035, df=3, p<.001).

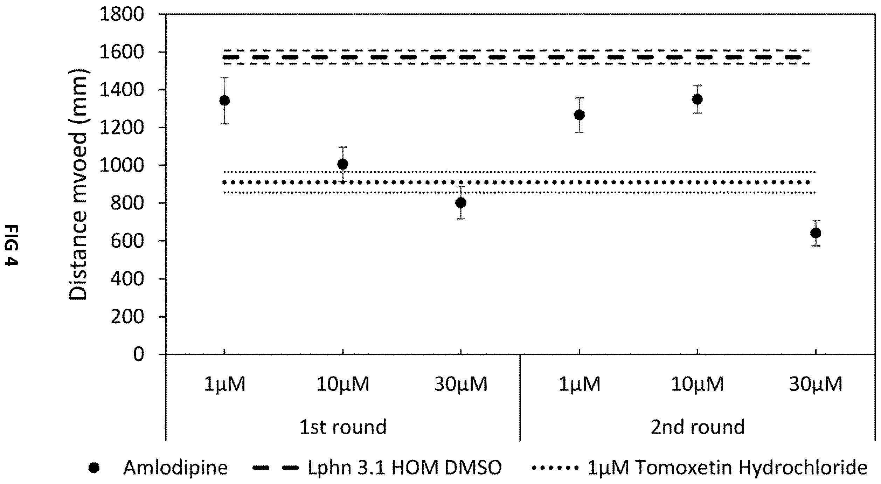

Figure 4 shows a comparison of the distance moved by Lphn3.1 HOM larvae that received amlodipine, atomoxetine (referred to as tomoxetin hydrochloride in Figure 4) or a vehicle control (i.e. DMSO only). The dashed line on the plot indicates the average distance moved by Lphn3.1 HOM larvae over five lights-on phases following treatment with the vehicle control (n=189). The dotted line indicates the average distance moved by Lphn3.1 HOM larvae over the five lights-on phases following treatment with 1 µM atomoxetine hydrochloride (n=24). The dots indicate the effect of amlodipine at a dosage of 1 µM, 10 µM or 30 µM on the average distance moved by Lphn3.1 HOM larvae over the five lights-on phases (n=24) (all data presented with +/- SEM). Statistically significant differences were found between larvae treated with amlodipine and larvae that received the vehicle control (f=23,684, df=3, p<.001, second round: f=31,699, df=3, p<.001).

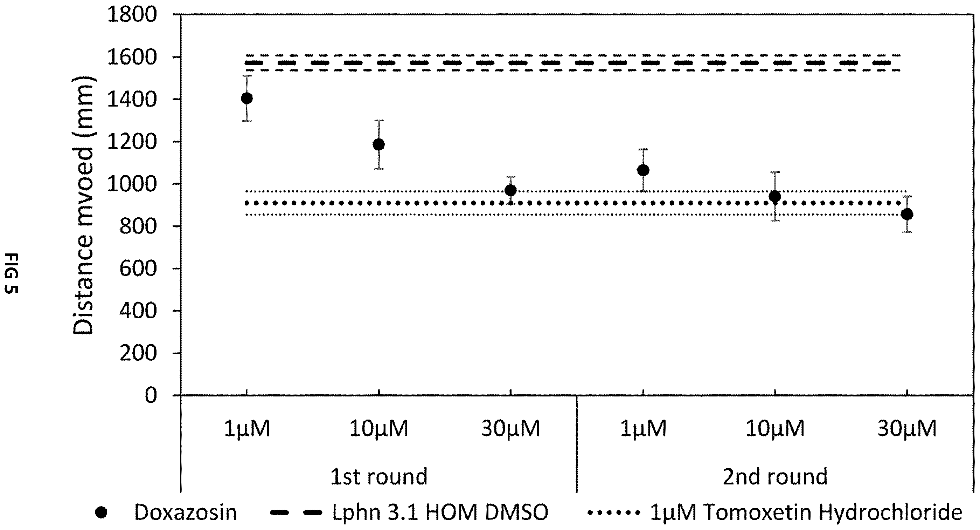

Figure 5 shows a comparison of the distance moved by Lphn3.1 HOM larvae that received doxazosin, atomoxetine (referred to as tomoxetin hydrochloride in Figure 5) or a vehicle control (i.e. DMSO only). The dashed line on the plot indicates the average distance moved by Lphn3.1 HOM larvae over five lights-on phases following treatment with the vehicle control (n=189). The dotted line indicates the average distance moved by Lphn3.1 HOM larvae over the five lights-on phases following treatment with 1 µM atomoxetine hydrochloride (n=24). The dots indicate the effect of doxazosin at a dosage of 1 µM, 10 µM or 30 µM on the average distance moved by Lphn3.1 HOM larvae over the five lights-on phases (n=24) (all data presented with +/- SEM). Statistically significant differences were found between larvae treated with doxazosin and larvae that received the vehicle control (f=14,642, df=3, p<.001, second round: f=29,29, df=3, p<.001).

### Detailed Description

The inventors of the present invention have found that the compound according to formula (II) is surprisingly effective for the treatment or prophylaxis of ADHD.

As discussed in more detail below, the present inventors have found that amlodipine (compound according to formula (II)) is surprisingly effective at reducing the activity of zebrafish larvae in a model of ADHD. The ADHD model used by the present inventors uses zebrafish carrying a homozygous knock-out of the *Latrophilin 3* (*lphn.3.1*) gene. Mutations in the corresponding *lphn.3.1* gene in humans (i.e. *LPHN3*) have been strongly implicated as a risk factor for ADHD in humans (Arcos-Burgos et al, 2010, Mol Psychiatry 15, 1053-1066, and Lange et al., Prog Neuropsychopharmacol Biol Psychiatry, 2018, 84(A), 181-189). The function of the *lphn.3.1* gene in zebrafish has been found to correlate with the function observed in humans. In the zebrafish model used by the present inventors, ADHD-like behavioural phenotypes, such as hyperactivity and motor impulsivity, are observed in zebrafish larvae carrying a homozygous knock-out of the *lphn3.1* gene. The present inventors have also identified, using the zebrafish model of ADHD, amlodipine, a dihydropyridine calcium channel blocker typically used to treat high blood pressure and angina, as being surprisingly effective at reducing ADHD behaviours such as hyperactivity and motor impulsivity.

Thus, the present invention provides the compound of formula (II) (amlodipine).. or a pharmaceutically acceptable salt or solvate thereof, for use in the treatment or prophylaxis of ADHD.

For the avoidance of doubt, in this document, it is intended that the compound of formula (II) includes all salts and solvates thereof, unless stated otherwise

The compound of formula (II) may be prepared using methods known to those skilled in the art of organic chemistry. Pharmaceutical preparations of the compound of formula (II) suitable for use in the present invention are available from commercial sources. For example, pharmaceutical preparations of the compound of formula (II) are marketed under the name ISTIN^{®}, and generic pharmaceutical preparations of the compound of formula (II) are also available.

For the avoidance of doubt, in this document, it is intended that the compounds of formula (II) include all enantiomers thereof, unless stated otherwise. Also for the avoidance of doubt, in this document, it is intended that the compounds of formula (II) include compounds that differ by immaterial structural variations, and therefore expected to display similar properties to amlodipine in the ADHD model described herein.

The compound of formula (II) may form salts or solvates. Salts of the compound of formula (II) which are suitable for use in the present invention are those wherein a counterion is pharmaceutically acceptable. However, the use of salts having non-pharmaceutically acceptable counter-ions are within the scope of the present invention, for example, for use as intermediates in the preparation of the compound of formula (II) and their pharmaceutically acceptable salts, and physiologically functional derivatives. Suitable salts for use according to the invention include those formed with organic or inorganic acids. In particular, suitable salts formed with acids for use according to the invention include those formed with mineral acids, strong organic carboxylic acids, such as alkanecarboxylic acids of 1 to 4 carbon atoms which are unsubstituted or substituted, for example, by halogen, such as saturated or unsaturated dicarboxylic acids, such as hydroxycarboxylic acids, such as amino acids, or with organic sulfonic acids, such as (C₁-C₄)-alkyl- or aryl-sulfonic acids which are unsubstituted or substituted, for example by halogen. Pharmaceutically acceptable acid addition salts include those formed from hydrochloric, hydrobromic, sulphuric, nitric, citric, tartaric, acetic, phosphoric, lactic, pyruvic, acetic, trifluoroacetic, succinic, perchloric, fumaric, maleic, glycolic, lactic, salicylic, oxalic, oxaloacetic, methanesulfonic, ethanesulfonic, p-toluenesulfonic, formic, benzoic, malonic, naphthalene-2-sulfonic, benzenesulfonic, isethionic, ascorbic, malic, phthalic, aspartic, and glutamic acids, lysine and arginine. Suitable cations which may be present in salts include alkali metal cations, especially sodium, potassium and calcium, and ammonium or amino cations.

Those skilled in the art of organic chemistry will appreciate that many organic compounds can form complexes with solvents in which they are reacted or from which they are precipitated or crystallized. These complexes are known as "solvates". For example, a complex with water is known as a "hydrate". The complex may incorporate a solvent in stoichiometric or non-stoichiometric amounts. Solvates are described in Water-Insoluble Drug Formulation, 2nd ed R. Lui CRC Press, page 553 and Byrn et al Pharm Res 12(7), 1995, 945-954. Before it is made up in solution, the compound of formula (II) may be in the form of a solvate. Solvates of the compound of formula (II) which are suitable for use as a medicament according to the invention are those wherein the associated solvent is pharmaceutically acceptable. For example, a hydrate is a pharmaceutically acceptable solvate.

A compound which, upon administration to the recipient, is capable of being converted into the compound of formula (II) or an active metabolite or residue thereof, is known as a "prodrug". Thus, in certain embodiments, the compound of formula (II) may be provided in the form of a prodrug. A prodrug may, for example, be converted within the body, e.g. by hydrolysis in the blood, into its active form that has medical effects. Pharmaceutical acceptable prodrugs are described in T. Higuchi and V. Stella, Prodrugs as Novel Delivery Systems, Vol. 14 of the A. C. S. Symposium Series (1976); "Design of Prodrugs" ed. H. Bundgaard, Elsevier, 1985; and in Edward B. Roche, ed., Bioreversible Carriers in Drug Design, American Pharmaceutical Association and Pergamon Press, 1987.

### Pharmaceutical compositions

While it is possible for the compound of formula (II) to be administered alone, it is preferable for it to be present in a composition and particularly in a pharmaceutical composition. Pharmaceutical compositions of the present invention comprise the compound of formula (II) and one or more pharmaceutically acceptable excipients.

Pharmaceutical compositions include those suitable for oral, parenteral (including subcutaneous, intradermal, intraosseous infusion, intramuscular, intravascular (bolus or infusion), and intramedullary), intraperitoneal, transmucosal, transdermal, rectal and topical (including dermal, buccal, sublingual and intraocular) administration, although the most suitable route may depend upon the characteristics of the subject under treatment, for example the species, age, weight, sex, medical conditions, the particular type of ADHD (for example, "impulsive type/hyperactive type", "inattentive type" and "combined type" ADHD) and its severity, and other relevant medical and physical factors.

Formulations for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilised) condition requiring only the addition of the sterile liquid carrier, for example saline or water-for-injection, immediately prior to use. Extemporaneous injection solutions and suspensions may be prepared from sterile powders, granules and tablets of the kind described herein. Exemplary compositions for parenteral administration include injectable solutions or suspensions which can contain, for example, suitable non-toxic, parenterally acceptable diluents or solvents, such as mannitol, 1,3-butanediol, water, Ringer's solution, an isotonic sodium chloride solution, or other suitable dispersing or wetting and suspending agents, including synthetic mono- or diglycerides, and fatty acids, including oleic acid, or Cremaphor. Compositions for nasal, aerosol or inhalation administration include solutions in saline, which can contain, for example, benzyl alcohol or other suitable preservatives, absorption promoters to enhance bioavailability, and/or other solubilizing or dispersing agents such as those known in the art.

Formulations for rectal administration may be presented as a suppository with carriers such as cocoa butter, synthetic glyceride esters or polyethylene glycol. Such carriers are typically solid at ordinary temperatures, but liquefy and/or dissolve in the rectal cavity to release the drug.

Formulations for topical administration in the mouth, for example buccally or sublingually, include lozenges comprising the active ingredient in a flavoured basis such as sucrose and acacia or tragacanth, and pastilles comprising the active ingredient in a basis such as gelatin and glycerine or sucrose and acacia. Exemplary compositions for topical administration include a topical carrier such as Plastibase (mineral oil gelled with polyethylene).

Pharmaceutical compositions suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil-in-water liquid emulsion or a water-in-oil liquid emulsion. The compound of formula (II) may also be presented as a bolus, electuary or paste. The pharmaceutical compositions may optionally be present in a form that provides slow or controlled release of the compound of formula (II) once administered to a subject. Various pharmaceutically acceptable carriers and their formulation are described in standard formulation treatises, e.g., Remington's Pharmaceutical Sciences by E. W. Martin. See also Wang, Y. J. and Hanson, M. A., Journal of Parenteral Science and Technology, Technical Report No. 10, Supp. 42:2S, 1988.

Preferred unit dosage compositions are those containing an exploratory dose or therapeutic dose, or an appropriate fraction thereof, of the compound of formula (II).

In preferred embodiments, a composition for use according to the present invention consists essentially of the compound of formula (II) and at least one pharmaceutically acceptable excipient.

An exemplary composition comprising the compound of formula (II) for use according to the present invention comprises one or more of the following pharmaceutically acceptable carriers: calcium hydrogen phosphate, microcrystalline cellulose, sodium starch glycolate, magnesium stearate, sodium acid citrate, sodium croscarmellose, crospovidone, anhydrous calcium hydrogen phosphate, anhydrous colloidal silica. A further exemplary composition comprising the compound of formula (II) for use according to the present invention is a tablet comprising: sodium starch glycolate, anhydrous calcium hydrogen phosphate, microcrystalline cellulose, magnesium stearate. A further exemplary composition comprising the compound of formula (II) for use according to the present invention is an oral solution comprising: methyl parahydroxybenzoate, propylene glycol, anhydrous disodium phosphate, sodium dihydrogen phosphate dihydrate, purified water, glycerol, maltitol liquid.

It should be understood that in addition to the ingredients particularly mentioned above, the compositions for use in this invention may include other agents conventional in the art having regard to the type of composition in question.

The compositions for use according to the invention may comprise one or more further therapeutic agents. Examples of further therapeutic agents that may be present in a composition for use according to the present invention include, but are not limited to, methylphenidate, amphetamine (for example dexamphetamine), lisdexamfetamine, atomoxetine, viloxazine, clonidine, and guanfacine. Typically, one or more further therapeutic agents is a stimulant, such as amphetamine, methylphenidate and lisdexamfetamine.

### Attention Deficit/Hyperactivity Disorder (ADHD)

The present inventors have found that the compound of formula (II) and pharmaceutical compositions thereof, is particularly effective at reducing the symptoms of ADHD in a subject known or suspected of having ADHD, or delaying the onset of, or preventing ADHD in a subject known or suspected of being at risk of developing ADHD.

Thus, the compound of formula (II)or a pharmaceutical composition thereof, for use according to the present invention, may be administered to a subject known or suspected of having ADHD, or known or suspected of being at risk of developing ADHD. The subject may be a human subject, for example a human patient. The human subject may be a child (e.g. under the age of about 10 years old), an adolescent (e.g. between the ages of about 10 to 19 years old) or may be an adult (e.g. over the age of about 18 to 21 years old).

The subject known or suspected of having ADHD may have ADHD that is characterised by impulsive and hyperactive behaviours without impaired levels of attention (i.e. "impulsive type/hyperactive type" ADHD). Or, the subject known or suspected of having ADHD may have ADHD that is characterised by impaired levels of attention without hyperactivity or impulsivity (i.e. "inattentive type" ADHD). Or, the subject known or suspected of having ADHD may have ADHD that is characterised by a combination of reduced levels of attention with hyperactivity and impulsivity behaviours (i.e. "combined type" ADHD).

A subject known or suspected of being at risk of developing ADHD may be one who has a known or suspected genetic predisposition for developing ADHD, for example, the subject may have a mutation in the *Latrophilin 3* (*LPHN3*) gene. Examples of further genetic mutations implicated in ADHD have been reported (see, for example, Faraone and Larsson, Molecular Psychiatry, 2019, 24, 562-575, which is incorporated herein by reference), and include, for example, mutations in one or more of the following genes: the *serotonin transporter* (*5HTT*) gene, the *dopamine transporter* (*DAT1*) gene, the *D4 dopamine receptor* (*DRD4*) gene, the *D5 dopamine receptor* (*DRD5*) gene, the serotonin 1B receptor gene (*HTRIB*) and the *Synaptosomal-Associated Protein (SNAP25)* gene. Additionally, or alternatively, a subject known or suspected of being at risk of developing ADHD may be one who has been exposed to one or more potential environmental risk factors associated with ADHD, such as maternal pre-pregnancy obesity, pre-eclampsia, hypertension, acetaminophen exposure, and smoking during pregnancy; and childhood atopic diseases. Additionally, the subject known or suspected of having ADHD, or known or suspected of being at risk of developing ADHD, may also have signs or symptoms of other conditions such as depression, anxiety disorder, oppositional defiant disorder (ODD), conduct disorder, sleeps problems (for example, sleep-onset insomnia), autism spectrum disorder (ASD), bipolar disorder, epilepsy, Tourette's syndrome and/or leaning difficulties such as dyslexia. Typically, the subject is one who meets the diagnostic criteria for ADHD set out in the Diagnostic and Statistical Manual of Mental Disorders (DSM) or International Classification of Diseases (ICD). Additionally, the subject known or suspected of having ADHD, or known or suspected of being at risk of developing ADHD, may also suffer from a substance abuse disorder, for example addiction and/or abuse of stimulants such as amphetamine.

For example, the compound of formula (II) or compositions thereof, for use according to the invention, may be administered to a subject for whom one or more established stimulant-based ADHD treatments (for example, methylphenidate, dexamphetamine, and lisdexamfetamine) are deemed to be unsuitable due to the subject being at risk, having a history of, or currently suffering from, a substance abuse disorder.

The present inventors have shown using a zebrafish model of ADHD that the compound of formula (II) is surprisingly effective at reducing hyperactivity and motor impulsivity. The efficacy of the compound of formula (II) at reducing hyperactivity and motor impulsivity in the zebrafish model of ADHD makes the compound of formula (II) an especially attractive treatment for ADHD that is characterised by hyperactivity and/or impulsivity behaviours (i.e. "impulsive type/hyperactive type" ADHD or "combined type" ADHD). Thus, in certain embodiments, the compound of formula (II) or a pharmaceutical composition thereof, for use according to the present invention, may be administered to a subject known or suspected of having ADHD that is characterised by impulsive and hyperactive behaviours without impaired levels of attention (i.e. "impulsive type/hyperactive type" ADHD), or ADHD that is characterised by a combination of reduced levels of attention with hyperactivity and impulsivity behaviours (i.e. "combined type" ADHD).

The compound of formula (II) or compositions thereof, for use according to the invention, may be administered to a subject for whom one or more established ADHD treatments (for example, methylphenidate, dexamphetamine, lisdexamfetamine, atomoxetine, viloxazine, guanfacine and clonidine) have been ineffective at reducing one or more of the symptoms of ADHD and/or induced intolerable adverse effects. Examples of adverse effects known or suspected of being caused by established ADHD treatments include impaired sleep, lack of appetite, increased blood pressure, stunted growth, disruption of circadian rhythm and neurotoxicity.

The compound of formula (II) and compositions thereof, as described herein finds utility in a method of treating or preventing ADHD, wherein said method comprises a step of administering the compound of formula (II) or a composition thereof, to a patient known or suspected of having ADHD, or known or suspected of being at risk of developing ADHD.

### Dosage regimens

The amount of the compound of formula (II) which is required to achieve a therapeutic effect will vary with the particular route of administration and the characteristics of the subject under treatment, for example the species, age, weight, sex, medical conditions, the particular type of ADHD (for example "impulsive type/hyperactive type", "inattentive type" and "combined type" ADHD) and its severity, and other relevant medical and physical factors. An ordinarily skilled physician can readily determine and administer an effective amount of the compound of formula (II) required for treatment or prophylaxis of ADHD

The compound of formula (II) may be administered daily (including several times daily), every second or third day, weekly, every second, third or fourth week or even as a single dose depending on the subject and the characteristics of the ADHD to be treated.

The compound of formula (II) (excluding the mass of any counterion or solvent) may be administered in an amount of about 0.1 mg to 10 mg per administration. For example, at least 0.1 mg, at least 0.5 mg, at least 1 mg, at least 5 mg, at least 2 mg, at least 2.5 mg, at least 3 mg, at least 4 mg, at least 5 mg, at least 6 mg, at least 7 mg, at least 8 mg, at least 9 mg, at least 10 mg may be administered to a subject. Typically, the compound of formula (II) is administered as a single daily dose of about 1 mg, 1.5 mg, 2 mg, 2.5 mg, 3 mg, 3.5 mg, 4 mg, 4.5 mg, 5 mg or 10 mg (excluding the mass of any counterion or solvent). For example, a single daily dose of 2 mg, 2.5 mg, 5 mg, 7.5 mg or 10 mg. Alternatively, the compound of formula (II) is administered as a dose of about 1 mg, 1.5 mg, 2 mg, 2.5 mg, 3 mg, 3.5 mg, 4 mg, 4.5 mg or 5 mg (excluding the mass of any counterion or solvent), two, three or four times a day. For example, a daily dose of 5 mg per day may be administered as two separate doses of 2.5 mg, wherein, for example, the first dose is administered in the morning and the second dose is administered in the evening (for example, after 6 p.m.). Or, for example, a daily dose of 10 mg per day may be administered as two separate doses, wherein, for example, a first dose of 5 mg is administered in the morning and the second dose of 5 mg is administered in the evening.

In certain embodiments, the compound of formula (II) is administered as a composition. Preferably, the composition is a pharmaceutical composition for use according to the present invention.

Whilst the compound of formula (II) may be used as the sole active ingredient in the present invention, it is also possible for it to be used in combination with one or more further therapeutic interventions, and the use of such combinations provides one embodiment of the present invention. Examples of further therapeutic interventions include pharmacological interventions, dietetic interventions and psychological intervention.

Further pharmacological interventions may be therapeutic agents useful in the treatment or prophylaxis of ADHD, or other pharmaceutically active materials. Such agents are known in the art. Examples of further therapeutic agents for use in the present invention include those described herein. Typically, the further therapeutic agent is a stimulant, such as amphetamine, methylphenidate and lisdexamfetamine. Examples of suitable dietetic interventions include, for example, supplementary fatty acids and the exclusion of artificial food colour from the diet. Examples of suitable psychological interventions include, for example, cognitive behavioural therapy (CBT).

The one or more further therapeutic interventions may be used simultaneously, sequentially or separately with/from the administration of the compound of formula (II). The individual components of such combinations can be administered separately at different times during the course of therapy or concurrently in divided or single combination forms. An ordinarily skilled physician can readily determine and administer the effective amount of one or more therapeutic interventions required to have the desired therapeutic effect.

Preferred unit dosage compositions for use according to the invention are those containing an effective dose, or an appropriate fraction thereof, of the compound of formula (II). The release of the compound of formula (II) from certain composition may also be sustained, for example, if the composition contains suitable controlled-release excipients.

### Kits

The present invention provides a kit comprising the compound of formula (II) one or more pharmaceutically acceptable excipients, and optionally one or more further therapeutic agents that are useful in the treatment or prophylaxis of ADHD. Examples of such further therapeutic agents include those described herein as being suitable for use in the present invention, and being optionally present in a pharmaceutical composition of the invention as a further therapeutic agent.

Kits of the present inventions may also contain instructions for a dietetic intervention and/or instructions for a psychological intervention suitable for ADHD. For example, the kits may include instructions for a nutrition plan suitable for the subject receiving treatment and/or the kit may comprise instructions/guidance for cognitive behavioural therapy (CBT).

Kits of the present invention find use in the treatment and prophylaxis of ADHD.

For the avoidance of doubt, the compound of formula (II) present in a kit according to the present invention is in a form and quantity suitable for use according to the present invention. Suitable pharmaceutical compositions and formulations are described herein. The skilled person can readily determine a quantity of the compound of formula (II) suitable for including in a kit of the invention, and for use according to the invention.

### Metabolites

Active metabolites of the compound of formula (II) may be used in the treatment or prophylaxis of Attention Deficit/Hyperactivity Disorder (ADHD). In embodiments wherein the compound is an active metabolite of a compound of formula (II) the compound may be isolated from cells treated with the compound of formula (II) or may be prepared using standard organic chemistry techniques. For the avoidance of doubt, when the compound for use according to the present invention is an active metabolite of a compound of formula the compound may be used in the form of a composition and/or as medicaments in the same manner as described herein for the compound of formula (II).

The following Examples illustrate the invention.

### Examples

*Danio rerio* is gaining popularity in biological psychiatry. Their rich behavioral repertoire, the availability of well-established behavioral and automated behavioral assays, make zebrafish a useful model of various human brain disorders. The neuronal pathways involved in brain physiology are highly conserved, including all major neurotransmitter systems and high genetic homology. Gene editing technology allows precise modelling of human disorders. The lphn3.1 knock-out model of ADHD described herein robustly exhibits the hallmarks of human ADHD, that is: hyperactivity, hypersensitivity to known dopamine agonists, and rescue of the phenotype following administration of known anti ADHD compounds.

### Material and methods:

Zebrafish larvae carrying a knock-out of the *Latrophilin3* (*Lphn3.1*) gene were generated by CRISPR-Cas9. Zebrafish were kept in a 14:10 light: dark cycle in 3 or 10 L multi tank constant flow system (Aquatic Habitats, Apopka, FL, USA). For behavioral analysis a total number of 881, 6 days-post fertilization (dpf) embryos carrying a homozygous knock-out of the *Lphn3.1* gene were used in the study. Adult zebrafish with a homozygous knock-out of the *Lphn3.1* gene and adult zebrafish with a wild-type *Lphn3.1* gene were fed three times a day on a variable diet of TetraMin flakes (Tetra Holding GmbH, Melle, Germany) and live Artemia. Water temperature was held at a constant 28.5 °C and replaced at a rate of 10% per day. Eggs were collected between 10:00-12:00 a.m. and contained in 2 L tanks. The following day, dead eggs were removed and tanks were cleaned. Eggs were incubated for 4 days at 28.5 °C in system water mixed with methylene blue. All procedures in the study were carried out in strict compliance with the regulations of, and approved by, the National Bioethics Committee of Iceland (regulation 460/2017).

### Genotyping

Strains were verified for knock-down of the *Lphn3.1* gene using Western blot.

### Behavioral Recordings

At 5 dpf, larvae were placed in individual wells of 96-microwell plates (Nunc, Roskilde, Denmark) in system water. The microwell plates were relocated to a custom-built activity monitoring system fitted with 24 infrared cameras (Ikegami, ICD-49E; Ikegami Tsushinki Co, Japan) which was thermo-regulated at 28,5°C, blocked from daylight and illuminated from below with white (255 lx; light-phase) and infrared light (0 lx; dark-phase). Larvae behavior was tracked in two dimensions at 5 Hz. Larvae were left to acclimatize in the activity monitoring system for 24 hours prior to recording. Exclusion criterion was based on the percentage of samples during a recording where a larva was not tracked. The threshold was set at 10%, thus a larva that was tracked <90% of the total recording time was excluded from the study.

### Motor Assay

Following a 24 hour acclimation period, behavioral recordings were made. Locomotor activity was recorded between 1.00 pm and 6.00 pm at 6 dpf during alternating light and dark conditions, presented in 30-minute intervals. For this period, average distance moved (mm) by the larvae was calculated as the mean of the total distance swum during five separate 30-minute intervals immediately after transition of light conditions.

**Example 1: Efficacy of aceclofenac,** (reference compound not encompassed by the invention), **amlodipine or doxazosin** (reference compound not encompassed by the invention), **compared to atomoxetine** (reference compound not encompassed by the invention), **in the zebrafish model of ADHD** Zebrafish larvae with a homozygous knock-out of the *Lphn3.1* gene (herein referred to as "Lphn3.1 HOM") and zebrafish larvae with a wild-type *Lphn3.1* gene (herein referred to as "Lphn3.1 WT") were exposed to the on-the-market ADHD drug atomoxetine hydrochloride (tomoxetine hydrochloride), aceclofenac (Prestwick, 67400 Illkirch, France), amlodipine (Prestwick, 67400 Illkirch, France), doxazosin (Prestwick, 67400 Illkirch, France), or a vehicle control before behavioral recordings started.

Drug preparation was performed on the day of recording. Drugs were diluted from stock solution using distilled water (Invitrogen, Paisley, PA4 9RF, UK). Three different concentration of each drug were used, 1 µM, 10 µM and 30 µM. Additionally, 0.03% DMSO (Sigma-Aldrich, St. Louis, USA) solution was prepared for the vehicle control group. Drugs and vehicle control were added into the microwells on the day of recording, between 11.30 a.m. and 12.30 p.m. Data was obtained using EthoVision XT (Version 11.5.2016, Noldus) and exported to Microsoft Excel for analysis. Statistical analysis was performed using IBM SPSS Statistics for Windows, version 26 (IBM corp., Armonk, N.Y., USA). Figures were produced using Microsoft Excel and GraphPad Prism Software (Version 5.01, GraphPad Software Inc.). Data are presented as mean ± standard error of the mean (s.e.m). For analysis of Lphn 3.1 naive (i.e. untreated larvae that do not receive a test compound or a vehicle control) and DMSO larvae (i.e. larvae receiving only the DMSO vehicle control), statistical differences were evaluated using two-way ANOVA. For analysis of Lphn 3.1 larvae treated with aceclofenac statistical differences were evaluated using one-way ANOVA with Dunnett two-sided post hoc analysis. *P<0.05* was considered statistically significant.

### Results:

Following 24 hours of recording it was evident that the Lphn3.1 HOM larvae display a notable hyperactive phenotype. Higher peak velocities following lights-off (which results in a stereotypical short, increase in activity for zebrafish), as well as higher overall average velocity during the lights-on periods, were observed for Lphn3.1 HOM larvae (Figure 1). In Figure 1, the activity of the Lphn3.1 HOM larvae is shown in the upper line and the activity of the Lphn3.1 WT larvae is shown in the lower line. The overall activity pattern of the larvae suggested that any period could be selected for statistical analysis.

Using the average distance moved during lights-on periods it was demonstrated, first, that homozygous larvae were indeed statistically significantly hypermotile compared to wild-types and, second, the vehicle (DMSO) had no effects on behavior (Figure 2).

Atomoxetine, aceclofenac, amlodipine, and doxazosin were each found to increase velocity in wild-type larvae. The effects of aceclofenac, amlodipine and doxazosin were compared to optimal dose of atomoxetine (see Figure 3, 4 and 5, respectively). Comparison with Lphn3.1 HOM larvae that were administered the vehicle control, revealed that all doses of aceclofenac, amlodipine, and doxazosin differed significantly from vehicle control and untreated larvae (in a dose dependent manner). These data show that aceclofenac, amlodipine, and doxazosin are effective at reducing ADHD-like phenotypes in Lphn3.1 HOM larvae.

## Claims

1. A compound according to formula (II): 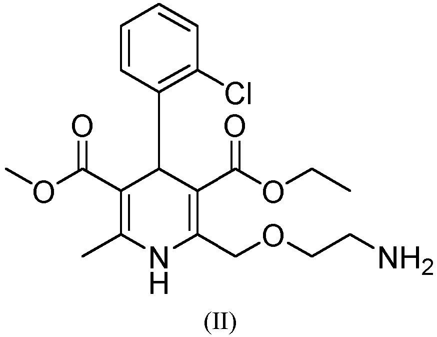
or a pharmaceutically acceptable salt or solvate thereof;
for use in the treatment or prophylaxis of Attention Deficit/Hyperactivity Disorder (ADHD).

2. The compound of formula (II) or a pharmaceutically acceptable salt or solvate thereof, for use according to claim 1, for administration to a subject known or suspected of having ADHD, or known or suspected of being at risk of developing ADHD.

3. The compound for use according to claim 2, wherein the subject known or suspected of having ADHD, or known or suspected of being at risk of developing ADHD, also has signs or symptoms of other conditions such as depression, anxiety disorder, oppositional defiant disorder (ODD), conduct disorder, sleeps problems (for example, sleep-onset insomnia), autism spectrum disorder (ASD), bipolar disorder, epilepsy, Tourette's syndrome and/or leaning difficulties such as dyslexia.

4. The compound for use according to any one of claims 1 to 3 wherein compound is administered to a subject for whom one or more established ADHD treatments (for example, methylphenidate, dexamphetamine, lisdexamfetamine, atomoxetine, viloxazine, guanfacine and clonidine) have been ineffective at reducing one or more of the symptoms of ADHD and/or induced intolerable adverse effects.

5. The compound for use according to claim 4, where the adverse effects include impaired sleep, lack of appetite, increased blood pressure, stunted growth, disruption of circadian rhythm and neurotoxicity.

6. The compound for use according to claim 1 to 5, wherein the compound is administered in a dose of about 0.1 to 10mg (excluding the mass of any counterion or solvent).

7. The compound for use according to any one of claims 5 or 6, wherein a dose of the compound is administered as a single daily dose of 2 mg, 2.5 mg, 5.0 mg, 7.5 mg or 10 mg (excluding the mass of any counterion or solvent).

8. The compound for use according to any one of claims 5 to 7, wherein a dose of the compound is administered at a dose of 1 mg, 1.5 mg, 2 mg, 2.5 mg, 3 mg, 3.5 mg, 4 mg, 4.5 mg or 5 mg (excluding the mass of any counterion or solvent), two, three or four times a day.

9. The compound for use according to any one of claims 1 to 5, wherein the compound is administered in a pharmaceutical composition.

10. The compound for use according to any one of claims 1 to 4, wherein the compound is administered simultaneously, sequentially or separately with one or more further therapeutic interventions, for example a dietetic intervention, psychological intervention and/or pharmacological intervention.

11. The compound for use according to claim 5, wherein the compound is administered simultaneously, sequentially or separately with one or more further pharmacological interventions selected from methylphenidate, dexamphetamine, lisdexamfetamine, atomoxetine and guanfacine.

12. The compound for use, according to any one of claims 1 to 11, wherein the ADHD is selected from "impulsive type/hyperactive type" ADHD, "inattentive type" ADHD and "combined type" ADHD.

13. A kit comprising the compound defined in claim 1 and one or more further pharmacological intervention, wherein the one or more further pharmacological intervention is selected from methylphenidate, amphetamine (for example dexamphetamine), lisdexamfetamine, atomoxetine, viloxazine, clonidine, and/or guanfacine.

14. The kit of claim 13 for use in the treatment or prophylaxis of ADHD.

## Patentansprüche

1. Eine Verbindung gemäß der Formel (II): 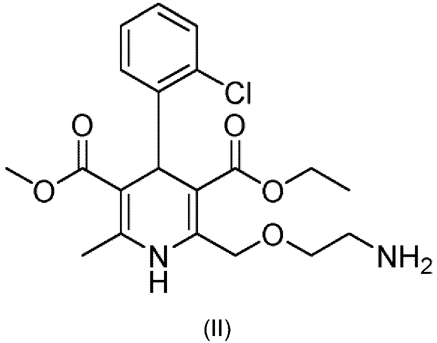
oder ein pharmazeutisch annehmbares Salz oder Solvat davon
zur Verwendung bei der Behandlung oder Prophylaxe von Aufmerksamkeitsdefizit-/ Hyperaktivitätsstörung (ADHS).

2. Die Verbindung der Formel (II) oder ein pharmazeutisch annehmbares Salz oder Solvat davon zur Verwendung gemäß Anspruch 1 zur Verabreichung an ein Subjekt, von dem bekannt ist oder vermutet wird, dass es an ADHS leidet, oder von dem bekannt ist oder vermutet wird, dass es ein Risikopotenzial für die Entwicklung von ADHS besitzt.

3. Die Verbindung zur Verwendung gemäß Anspruch 2, wobei das Subjekt, von dem bekannt ist oder vermutet wird, dass es an ADHS leidet, oder von dem bekannt ist oder vermutet wird, dass es ein Risikopotenzial für die Entwicklung von ADHS besitzt, auch Anzeichen oder Symptome anderer Zustände wie beispielweise Depression, Angststörung, oppositionelle Verhaltensstörung (ODD), Verhaltensstörung, Schlafstörungen (zum Beispiel Einschlafstörung), Autismus-Spektrum-Störung (ASS), bipolare Störung, Epilepsie, Tourette-Syndrom und/oder Lernschwierigkeiten wie z.B. Legasthenie besitzt.

4. Die Verbindung zur Verwendung gemäß einem der Ansprüche 1 bis 3, wobei die Verbindung an ein Subjekt verabreicht wird, bei dem eine oder mehrere herkömmliche ADHS-Behandlungen (zum Beispiel Methylphenidat, Dexamphetamin, Lisdexamfetamin, Atomoxetin, Viloxazin, Guanfacin und Clonidin) zur Verringerung von einem oder mehreren der Symptome von ADHS unwirksam waren und/oder unannehmbare unerwünschte Wirkungen verursachten.

5. Die Verbindung zur Verwendung gemäß Anspruch 4, wobei die unerwünschten Wirkungen Schlafbeeinträchtigung, Appetitlosigkeit, erhöhter Blutdruck, Kleinwuchs, Störungen des zirkadianen Rhythmus und Neurotoxizität umfassen.

6. Die Verbindung zur Verwendung gemäß Anspruch 1 bis 5, wobei die Verbindung in einer Dosis von etwa 0,1 bis 10 mg (wobei die Masse von Gegenionen oder Lösungsmittel nicht berücksichtigt wird) verabreicht wird.

7. Die Verbindung zur Verwendung gemäß einem der Ansprüche 5 bis 6, wobei eine Dosis der Verbindung als tägliche Einzeldosis von 2 mg, 2,5 mg, 5,0 mg, 7,5 mg oder 10 mg (wobei die Masse von Gegenionen oder Lösungsmittel nicht berücksichtigt wird) verabreicht wird.

8. Die Verbindung zur Verwendung gemäß einem der Ansprüche 5 bis 7, wobei eine Dosis der Verbindung in einer Dosis von 1 mg, 1,5 mg, 2 mg, 2,5 mg, 3 mg, 3,5 mg, 4 mg, 4,5 mg oder 5 mg (wobei die Masse von Gegenionen oder Lösungsmittel nicht berücksichtigt wird) zwei-, drei- oder viermal pro Tag verabreicht wird.

9. Die Verbindung zur Verwendung gemäß einem der Ansprüche 1 bis 5, wobei die Verbindung in einer pharmazeutischen Zusammensetzung verabreicht wird.

10. Die Verbindung zur Verwendung gemäß einem der Ansprüche 1 bis 4, wobei die Verbindung gleichzeitig, der Reihe nach oder separat mit einer oder mehreren weiteren therapeutischen Interventionen verabreicht wird, zum Beispiel einer diätischen Intervention, einer psychologischen Intervention und/oder einer pharmakologischen Intervention.

11. Die Verbindung zur Verwendung gemäß Anspruch 5, wobei die Verbindung gleichzeitig, der Reihe nach oder separat mit einer oder mehreren weiteren pharmakologischen Interventionen, ausgewählt aus Methylphenidat, Dexamphetamin, Lisdexamfetamin, Atomoxetin und Guanfacin, verabreicht wird.

12. Die Verbindung zur Verwendung gemäß einem der Ansprüche 1 bis 11, wobei die ADHS ausgewählt ist aus ADHS vom "hyperaktiv-impulsiven Typ", ADHS vom "Unaufmerksamkeitstyp" und ADHS vom "Kombinationstyp".

13. Ein Kit, der die in Anspruch 1 definierte Verbindung und eine oder mehrere weitere pharmakologische Interventionen umfasst, wobei die eine oder die mehreren weiteren pharmakologischen Interventionen ausgewählt sind aus Methylphenidat, Amphetamin (zum Beispiel Dexamphetamin), Lisdexamfetamin, Atomoxetin, Viloxazin, Clonidin und/oder Guanfacin.

14. Der Kit nach Anspruch 13 zur Verwendung bei der Behandlung oder Prophylaxe von ADHS.

## Revendications

1. Composé selon la formule (II) : 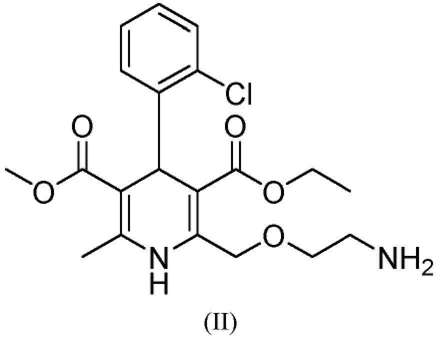
ou un sel ou solvate pharmaceutiquement acceptable de celui-ci,
pour utilisation dans le traitement ou la prophylaxie du trouble du déficit de l'attention/hyperactivité (TDAH).

2. Composé de formule (II) ou sel ou solvate pharmaceutiquement acceptable de celui-ci, pour utilisation selon la revendication 1, pour administration à un sujet connu ou suspecté d'avoir un TDAH, ou connu ou suspecté d'être à risque de développer un TDAH.

3. Composé pour utilisation selon la revendication 2, dans lequel le sujet connu ou suspecté d'avoir un TDAH, ou connu ou suspecté d'être à risque de développer un TDAH, présente également des signes ou des symptômes d'autres affections telles qu'une dépression, un trouble anxieux, un trouble oppositionnel avec provocation (TOP), un trouble du comportement, des problèmes de sommeil (par exemple, insomnie d'endormissement), un trouble du spectre autistique (TSA), un trouble bipolaire, l'épilepsie, le syndrome de Gilles de la Tourette et/ou des difficultés d'apprentissage telles que la dyslexie.

4. Composé pour utilisation selon l'une quelconque des revendications 1 à 3, dans lequel le composé est administré à un sujet pour lequel un ou plusieurs traitements établis du TDAH (par exemple, méthylphénidate, dexamphétamine, lisdexamfétamine, atomoxétine, viloxazine, guanfacine et clonidine) ont été inefficaces pour réduire un ou plusieurs des symptômes du TDAH et/ou ont induit des effets indésirables intolérables.

5. Composé pour utilisation selon la revendication 4, dans lequel les effets indésirables incluent une altération du sommeil, un manque d'appétit, une augmentation de la pression artérielle, un retard de croissance, une perturbation du rythme circadien et une neurotoxicité.

6. Composé pour utilisation selon les revendications 1 à 5, dans lequel le composé est administré à une dose d'environ 0,1 à 10 mg (à l'exclusion de la masse de tout contre-ion ou solvant).

7. Composé pour utilisation selon l'une quelconque des revendications 5 ou 6, dans lequel une dose du composé est administrée sous la forme d'une dose quotidienne unique de 2 mg, 2,5 mg, 5,0 mg, 7,5 mg ou 10 mg (à l'exclusion de la masse de tout contre-ion ou solvant).

8. Composé pour utilisation selon l'une quelconque des revendications 5 à 7, dans lequel une dose du composé est administrée à une dose de 1 mg, 1,5 mg, 2 mg, 2,5 mg, 3 mg, 3,5 mg, 4 mg, 4,5 mg ou 5 mg (à l'exclusion de la masse de tout contre-ion ou solvant), deux, trois ou quatre fois par jour.

9. Composé pour utilisation selon l'une quelconque des revendications 1 à 5, dans lequel le composé est administré dans une composition pharmaceutique.

10. Composé pour utilisation selon l'une quelconque des revendications 1 à 4, dans lequel le composé est administré simultanément, séquentiellement ou séparément avec une ou plusieurs autres interventions thérapeutiques, par exemple une intervention diététique, une intervention psychologique et/ou une intervention pharmacologique.

11. Composé pour utilisation selon la revendication 5, dans lequel le composé est administré simultanément, séquentiellement ou séparément avec une ou plusieurs autres interventions pharmacologiques choisies parmi méthylphénidate, dexamphétamine, lisdexamfétamine, atomoxétine et guanfacine.

12. Composé pour utilisation selon l'une quelconque des revendications 1 à 11, dans lequel le TDAH est choisi parmi le TDAH de « type impulsif/type hyperactif », le TDAH de « type inattentif » et le TDAH de « type combiné ».

13. Kit comprenant le composé défini dans la revendication 1 et une ou plusieurs autres interventions pharmacologiques, dans lequel les une ou plusieurs autres interventions pharmacologiques sont choisies parmi méthylphénidate, amphétamine (par exemple dexamphétamine), lisdexamfétamine, atomoxétine, viloxazine, clonidine et/ou guanfacine.

14. Kit selon la revendication 13, pour utilisation dans le traitement ou la prophylaxie du TDAH.
